# NEUE EUROPÄISCHE PATENTSCHRIFT

(11) **EP 2 356 734 B2**
(45) Veröffentlichungstag und Bekanntmachung des Hinweises auf die Entscheidung über den Einspruch: **12.01.2022**
(45) Hinweis auf die Patenterteilung: 16.09.2015
(21) Anmeldenummer: 09765015.4
(22) Anmeldetag: 13.11.2009
(51) Int. Cl.: H02K 1/14

(54) **SEGMENTIERTE STATOR-/ROTORELEMENTE VON ELEKTROMOTOREN**
SEGMENTED STATOR/ROTOR ELEMENTS OF ELECTRIC MOTORS
ÉLÉMENTS DE STATOR/ROTOR SEGMENTÉS POUR MOTEURS ÉLECTRIQUES

(30) Priorität: 14.11.2008 DE 102008057390
(43) Veröffentlichungstag der Anmeldung: 17.08.2011
(73) Patentinhaber: Robert Bosch GmbH, 70442 Stuttgart (DE)
(72) Erfinder: HACK, Arno, 97776 Eußenheim (DE); BORNER, Rudolf, 97854 Steinfeld (DE)
(86) Internationale Anmeldenummer: PCT/EP2009/008082
(87) Internationale Veröffentlichungsnummer: WO 2010/054824

(56) Entgegenhaltungen:
- EP-A1- 1 322 022
- EP-A2- 1 528 655
- WO-A1-98/32211
- WO-A1-2005/060073
- WO-A2-2008/075506
- DE-A1- 3 311 852
- DE-A1-102005 051 506
- DE-A1-102006 009 440
- US-A- 3 122 667
- US-A- 4 893 041
- US-A- 5 918 360
- US-A1- 2006 028 092
- US-A1- 2006 232 143
- US-A1- 2008 256 783
- US-B1- 6 265 804

## Beschreibung

### Beschreibung

Die Erfindung bezieht sich auf Elektromotoren und insbesondere auf Stator- und Rotorzähne von Elektromotoren, wobei die Elektromotoren als Servomotoren ausgeführt sein können. Derartige Stator- und Rotorzähne sind in vielfältigen Ausführungsformen aus dem Stand der Technik bekannt.

Dokument US 2006/0232143A1 zeigt beispielsweise einen vergossenen Stator.

Dokument EP 132 202 2 A1 zeigt einen Stator für einen Elektromotor mit einer Vielzahl von Polschenkeln und einem die Polschenkel umfänglich umgebenden Polring, bei welchem die Polschenkel jeweils aus jeweils zumindest zwei vorgefertigten Polschenkelelementen gebildet sind, welche jeweils eine Vielzahl aufeinandergeschichteter Polbleche aufweisen.

Dokument WO 98/32211 offenbart ein Verfahren zur Montage eines Ständers vor Ort und Dokument US 2008/0256783 offenbart ein Verfahren zum Herstellen einer Statoranordnung.

Dokument US 5918360 betrifft ein Verfahren zur Herstellung eines Schenkelpolmotors mit elektronischem Kommutator.

Dokument US 3122667 betrifft einen beschichteten Magnetkern zur Verwendung in elektrischen Induktionsmaschinen.

Dokument DE 102 006 009 440 A1 befasst sich mit der frühzeitigen Einbringung von Dauermagneten während eines Fertigungsprozesses.

Dokument EP 152 865 5 A2 betrifft einen speziell geformte Motoranordnung mit einer Vielzahl von isolierten Zähnen zur Aufnahme von Wicklungen.

Dokument US 626 580 4 B1 betrifft einen Motor und ein Verfahren zu dessen Herstellung.

Dokument WO 2008/075506 A2 einen Stator und ein Verfahren zu dessen Herstellung.

Die US 2006/0028092 A2 beschreibt einen Elektromotor mit Stator und Rotor, bei dem ein Statorzahn mit Endstücken verstehen und mittels einer Kunststofffolie isoliert ist.

Die aus dem Stand der Technik bekannten Elektromotoren verfügen über Statorzähne, die einteilig ausgeführt sind und die mit Isolierpapier bedeckt sind. Dieses Isolierpapier wird benötigt, um eine elektrische Isolierung zwischen den Kupferwicklungen der Spule und dem elektrisch leitenden Basiskörper des Zahnes herzustellen. Ferner bewirkt dieses Isolierpapier neben der elektrischen Isolierung auch eine thermische Isolierung, wodurch die Wärmeabfuhr des Motors behindert wird.

Um dies zu umgehen, wird mit Hilfe eines Spritzgießwerkzeuges eine Kunststoffschicht auf den Basiskörper aufgetragen, die eine bessere thermische Leitfähigkeit bei gleicher elektrischer Isolation ermöglicht. Das Aufbringen der Kunststoffschicht auf den Basiskörper erfolgt mit Hilfe von Spritzgießwerkzeugen. Ab Segmentlängen die größer als 100 mm sind, ergibt sich ein schwieriger bzw. nicht prozesssicherer Spritzvorgang, zudem entsteht ein erhöhtes technisches Risiko an den benötigten Bindenähten (elektrische Durchschlagfestigkeit, mechanische Stabilität). Ferner muss für jede Segmentbaulänge ein Spritzwerkzeug verwendet werden, wodurch höhe Kosten entstehen.

Der vorliegenden Erfindung liegt daher die Aufgabe zugrunde, eine kostengünstige Herstellung von Elektromotoren mit unterschiedlichen Statorzahnlängen zu ermöglichen, wobei für die Herstellung von Elektromotoren einer Motorbaugröße mit unterschiedlichen Motorbaulängen, d.h. mit verschiedenen Segmentbaulängen bevorzugt nur ein Spritzgießwerkzeug benötigt werden soll. Daneben sollen die notwendige Spritzlängen verringert und dadurch die Anforderungen an die Genauigkeit der Bauteile und den Spritzprozess erniedrigt werden.

Diese Aufgaben werden durch die Gegenstände der unabhängigen Ansprüche gelöst. Vorteilhafte Ausführungsformen und Weiterbildungen sind Gegenstand der Unteransprüche.

Ein erfindungsgemäßer Elektromotor weist wenigstens einen Stator und einen Rotor auf, wobei der Stator mindestens einen Statorzahn aufweist. Der wenigstens eine Statorzahn, weist mindestens ein erstes und ein zweites Endstück wie auch mindestens einen die Endstücke verbindenden Verbindungskörper zwischen dem ersten und dem zweiten Endstück auf. Weiterhin weist ein Verbindungskörper mindestens einen ersten Abschnitt als Anlagefläche für einen Teil einer Spule auf. Erfindungsgemäß ist der Verbindungskörper mehrteilig ausgebildet und besteht aus mindestens zwei Segmenten.

Durch eine derartige Anordnung erreicht man eine Verringerung der notwendigen Spritzlänge und damit wesentlich geringere Anforderungen an die Genauigkeit der Fertigungstechniken und der Bauteile. Dies vereinfacht den Spritzprozess, verringert den Ausschussanteil und senkt damit die Herstellkosten.

Besonders bevorzugt sind Elektromotoren mit 12 bzw. 18 Statorzähnen. Dies bedeutet jedoch nicht, dass die Erfindung lediglich auf Elektromotoren mit dieser Statoranzahl angewandt werden kann. Weiterhin bevorzugt sind auch Elektromotoren mit mehr bzw. weniger Zähnen sowie Elektromotoren mit einer ungeraden Anzahl an Statorzähnen.

Die Endstücke bestehen erfindungsgemäß aus Kunststoff und wirken daher elektrisch isolierend. Erfindungsgemäß ist wenigstens ein erster Abschnitt des Verbindungskörpers zumindest teilweise kunststoffumspritzt, dieser kunststoffumspritzte erste Abschnitt wirkt elektrisch isolierend und ist daher bevorzugt ein elektrisch isolierter Abschnitt.

Gegenüber dem Stand der Technik, in dem Isolierpapier eingesetzt wurde, wird durch die aufgespritzte Kunststoffschicht eine vergleichbare elektrische Isolation erhalten. Hierbei wird eine elektrische Isolation von mindestens 500 Volt und besonders bevorzugt von über 800 Volt erreicht. Erfindungsgemäß ist jedoch eine wesentlich bessere thermische Leitfähigkeit durch die Kunststoffschicht gegeben (Isolierpapier ca. 0,2 W/K*m, Kunststoff ca. 1,0 W/K*m). Es ist weiter bevorzugt, dass sich das Verhältnis der Wärmeleitfähigkeit von Isolierpapier zu dem eingesetzten Kunststoff von 1:5, zu 1:7,5 oder bevorzugt zu 1:10 verändert. In anderen Worten: Es ist erwünscht, dass die Kunststoffschicht eine hohe Wärmeleitfähigkeit aufweist, im weiteren Ausführungsformen werden daher Kunststoffschichten angestrebt, deren Wärmeleitfähigkeit bei beispielsweise 1,5 W/K*m bzw. 2 W/K*m oder höher liegt.

In einer weiteren bevorzugten Ausführungsform der Erfindung sind die mindestens zwei Segmente im Wesentlichen gleichartig ausgebildet. Hierdurch ergibt sich der Vorteil, dass durch eine Vielzahl von Segmenten, die aneinander angeordnet werden können, das Verbindungselement bzw. der erste Abschnitt um eine beliebige Länge verlängert werden kann.

Weiterhin ist es bevorzugt, dass mindestens zwei unterschiedlich lange Segmente pro Motorbaugröße verwendet werden. So kann beispielsweise ein variable Baukastensystem für jede Motorbaugröße entstehen mit dessen Hilfe jede gewünschte Segmentbaulänge, durch die Kombination verschieden langer Segmente bzw. gleichlanger Segmente, ermöglicht wird. Hierdurch lässt sich eine Herstellung einer elektrisch isolierten (bevorzugt über 700 Volt) und mechanisch stabilen Verbindung von zwei oder mehr kunststoffumspritzten Stator- und Rotorsegmenten, deren Baulänge von der Magnetteilung vorgegeben werden kann, erhalten. Idealerweise können Stator- und Rotorsegmente in beliebiger Länge gesteckt werden.

Ein weiterer Vorteil des durch Segmente zusammengefügten Verbindungskörpers resultiert aus einer Verringerung der verfahrenstechnisch benötigten Kunststoffdicke, wodurch ein höherer Kupferfüllfaktor innerhalb des Motors ermöglicht wird, was zu einer Leistungssteigerung des Motors führt. Weiter resultiert eine Erhöhung der Bauteilstabilität bei kürzeren Stanzpaketen bzw. Segmenten, da die alleine durch das Eigengewicht erzeugten Kräfte und Momente, die auf den Körper wirken, geringer sind und zudem besser abgestützt werden.

Ein weiterer Vorteil der bereits genannten Gegenstände der Erfindung ist, dass, wie bereits angedeutet, sich geringere Werkzeugkosten beim Umspritzen ergeben. Als weiteren Grund hierfür ist das einfachere Handling der kurzen Teile beim Umspritzen anzusehen. Die zuvor beschriebene Verringerung der benötigten Kunststoffdicke lässt sich zudem durch eine höhere Genauigkeit beim Spritzprozess (Parallelität und Rückfederkraft sind bei kurzen Segmenten wesentlich besser) erreichen. Ferner ist eine geringere Anzahl von Varianten anstelle einer Vielzahl von: "A, B, C, D, E - Längen" notwendig, da nur eine Grundlänge eines Segmentes und ein Endstück benötigt werden, wodurch sich die Anzahl der Gleichteile erhöht, dies führt zu nachgelagerten Vorteilen bei der Bestellung bzw. Lagerung von Materialien und Rohstoffen.

In einer weiteren bevorzugten Ausführungsform der vorliegenden Erfindung sind die Segmente zumindest in ihrer Länge verschieden.

Als ein Segment wird ein einzelner Körper verstanden, als zwei Segmente werden zwei einzelne Körper verstanden, usw. Hierbei ist es möglich, die Segmente durch Stecken, Kleben, Schweißen wie auch durch andere bekannte Fertigungs- und Fügetechniken zu verbinden. Beide Segmente haben bevorzugt den gleichen Querschnitt. Ferner bedeutet gleichartig, dass sie im Wesentlichen die gleiche Form aufweisen, wobei die Größe der einzelnen Elemente um einen Faktor x voneinander verschieden sein kann. In der Kontaktstelle in der sich die einzelnen Segmente berühren, weisen die Segmente jedoch einen im Wesentlichen kontinuierlichen Übergang auf.

Die Länge eines Statorzahnes erstreckt sich, wie auch die Länge der Segmente, in Richtung der Drehachse des Motors. Je längerdie Statorzähne ausgebildet sind, desto länger kann der Rotor ausgeführt sein, woraus eine Zunahme der Motorleistung resultiert.

In einer weiteren bevorzugten Ausführungsform der vorliegenden Erfindung sind die Segmente unlösbar miteinander verbunden. Eine unlösbare Verbindung führt dazu, dass die Segmente äußerst stabil miteinander verbunden sind und keine Relativbewegungen zwischen den Segmenten auftreten können. Hierdurch werden quasi einstückige Statorzähne geschaffen ohne, dass die Nachteile einstückig ausgebildeter Statorzähne auftreten.

Ferner ist es bevorzugt, dass die Segmente lösbar miteinander verbunden sind. Hierdurch ergeben sich Vorteile hinsichtlich der Demontage, wodurch Modifikationen, Recycling oder Wartungsarbeiten erleichtert bzw. ermöglicht werden.

In einer weiteren bevorzugten Ausführungsform der vorliegenden Erfindung ist je ein Endstück unlösbar mit einem Segment verbunden. Dies führt wiederum, wie bei der unlösbaren Verbindung zwischen den einzelnen Segmenten, auch hierzu einer möglichst stabilen Anordnung.

In einer weiteren bevorzugten Ausführungsform der vorliegenden Erfindung weist ein erster Abschnitt eines Statorzahnes eine Länge (L) von mindestens 10 mm, bevorzugt von 50-90 mm und besonders bevorzugt eine Länge in Abhängigkeit eines vielfachen der Magnetlänge, auf.

Je länger die Statorzähnen, desto länger kann der Rotor ausgeführt sein, woraus eine Zunahme der Motorleistung resultiert.

In einer weiteren bevorzugten Ausführungsform weist der erste Abschnitt eine Kunststoffschicht auf, deren Dicke unter 0,5 mm, bevorzugt unter 0,4 mm und besonders bevorzugt unter 0,3 mm liegt. Je dünner die Schicht, desto höher kann der Kupferfüllfaktor sein, wodurch die Leistung des Motors verbessert wird.

In einer weiteren bevorzugten Ausführungsform der vorliegenden Erfindung ist mindestens ein Teil eines zweiten Abschnittes und/oder mindestens ein Teil eines dritten Abschnittes elektrisch isolierend ausgestaltet. Als zweiter Abschnitt wird hierbei ein Bereich des Statorzahnfußes bezeichnet, der an den ersten Abschnitt angrenzt, und als dritter Abschnitt wird hierbei ein Bereich des Statorzahnkopfes bezeichnet, der auch an den ersten Abschnitt angrenzt. Durch die isolierte Ausgestaltung des zweiten und/oder des dritten Bereiches wird ein Kurzschluss zwischen einem bzw. beiden Bereich/en und der Spule vermieden.

Der zweite und/oder der dritte Bereich können eben oder kurvig ausgestaltet sein. Ferner können der erste und/oder der zweite Abschnitt zu dem ersten Abschnitt geneigt oder im rechten Winkel angeordnet sein.

Nach einer weiteren bevorzugten Ausführungsform sind innerhalb der Segmente magnetisierbare Körper angeordnet. Als magnetisierbare Körper werden hierbei insbesondere Eisenkerne verwendet. Es können allerdings auch alle weiteren magnetisierbaren Materialien eingesetzt werden.

Nach einer weiteren bevorzugten Ausführungsform der vorliegenden Erfindung entspricht der Querschnitt der Endstücke im Wesentlichen dem Querschnitt der Segmente. Dies bedeutet, dass die Endstücke gleichartige Querschnitte aufweisen. Bei einem Statorzahnaufbau aus zwei gleichartigen Endstücken und gleichartigen Segmenten wird so die Gleichteilanzahl erhöht, woraus wiederum die zuvor genannten Vorteile resultieren.

Ferner sind Endstücke verwendbar, deren Querschnitt im Wesentlichen von dem Querschnitt der Segmente verschieden sind. Wesentlich ist hierbei zum einen, dass die Wicklungen der Spule an der für sie vorgesehenen Stelle angeordnet werden können. Zum anderen ist wesentlich, dass zwischen der Spule und dem Statorzahn eine ständige bzw. durchgängige elektrische Isolation vorgesehen ist. Dies bedeutet, dass ein Kontakt zwischen der Spule und dem elektrisch leitenden Material des Statorzahnes vermieden werden muss, damit kein Kurzschluss auftritt.

Die vorliegende Erfindung bezieht sich weiterhin auf ein Verfahren zum Herstellen von Statorzähnen zur Anwendung in Elektromotoren gemäß Anspruch 8. Hierbei weist ein Statorzahn einen ersten Abschnitt auf, der erfindungsgemäß aus mindestens zwei Segmenten hergestellt wird, die in Längsrichtung eines Statorzahnes angeordnet werden. Hierbei werden die Segmente von einem ersten und einem zweiten Endteil in Längsrichtung abgeschlossen. Die Längsrichtung erstreckt sich auch, wie bereits zuvor beschrieben, in die Richtung der Drehachse.

Die Endteile sind hierbei an zueinander ausgerichteten Flächen angeordnet und sind in entgegengesetzte Richtungen orientiert.

In einer Ausführungsform werden Statorzähne für den Einsatz in Elektromotoren bereitgestellt. Hierbei weisen die Statorzähne einen metallischen Basiskörper auf. Es sind mindestens zwei Segmente in einem Verbindungskörper des Statorzahnes angeordnet, wodurch sich eine Länge des Statorzahnes von mindestens 10 mm ergibt. Hierbei sind weiterhin zwei Endstücke derart angeordnet, dass an mindestens zwei Seiten eines jeden Segmentes ein Endstück oder ein Segment angeordnet ist.

Der Basiskörper besteht aus Blech, das in definierten Abmessungen ausgestanzt wird. In Längenbereichen, die kleiner als 100 mm sind, kann beim Auftragen des Kunststoffes auf das Blech durch Kunststoffspritzen eine Dicke der Kunststoffschicht von <0,5mm, wie zuvor angedeutet, erreicht werden.
Weitere Vorteile und Ausführungsformen ergeben sich aus den beigefügten Zeichnungen.

Darin zeigen:
- Figur 1: Darstellung einer möglichen Anordnung der Statorzähne;
- Figur 2: perspektivische Darstellung eines Statorzahnes;
- Figur 3: Verbindungsstück eines Statorzahnes;
- Figur 4: Schnittzeichnung eines Statorzahnes;
- Figur 5a: mehrteilig zusammengesetzter Stator (Seitenansicht);
- Figur 5b: mehrteilig zusammengesetzter Stator (perspektivische Ansicht);
- Figur 5c: Detailansicht einer Verbindungsstelle;
- Figur 6: eine Darstellung der Komponenten aus denen ein segmentierter Statorzahn zusammengesetzt ist.

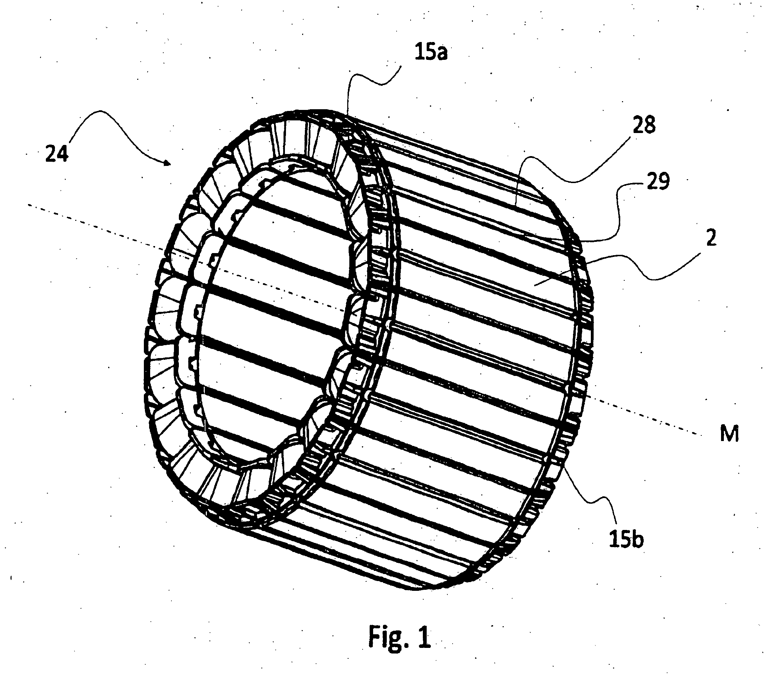

Figur 1 zeigt eine perspektivische Darstellung eines Stators 24. Der dargestellte Stator 24 besteht aus einer Vielzahl nebeneinander angeordneter Statorzähne 2. Ferner sind aus der

Darstellung neben den Statorzähnen 2 auch die Endstücke 15a, 15b ersichtlich, sowie die Verbindungselemente 28, 29 durch welche die Statorzähne in Kontakt stehen.

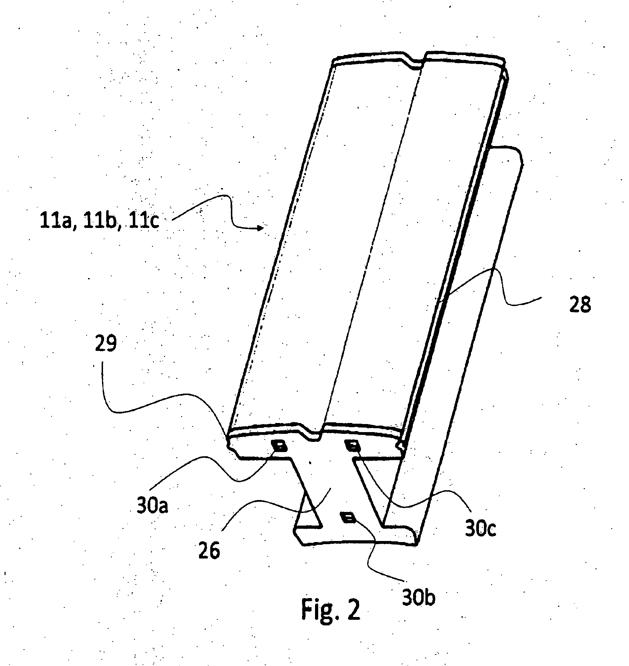

Figur 2 zeigt eine perspektivische Darstellung eines Statorsegmentes 11a, 11b, 11 c. Ferner geht aus der Darstellung der Kontaktbereich 26 hervor an dem ein weiteres Statorsegment 11 b oder ein bzw. zwei Endstücke 15a, 15b angeordnet werden können. Im von der Mit-telachse M des Motors weg orientierten Statorkopf sind Verbindungselemente 28, 29 zur Herstellung einer Verbindung zwischen den Statorzähnen 2 vorgesehen. Ferner sind die Kupplungselemente innerhalb des Kontaktbereiches 26 gezeigt.

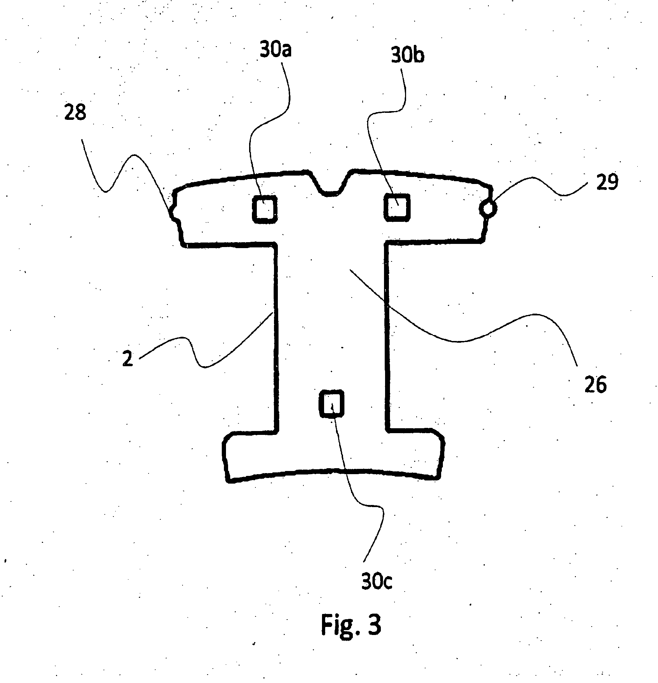

Figur 3 zeigt eine Vorderansicht eines Statorzahnsegmentes 11 a, 11 b, 11c. Aus der Darstellung gehen die Verbindungselemente 28, 29 und die Kopplungselemente 30a, 30b, 30c, die zur Herstellung einer Verbindung zwischen zwei Statorzahnsegmenten 11a, 11b, 11 c oder zwischen Statorzahnsegmenten 11 a, 11b, 11c und Endstücken 15a, 15b verwendet werden, hervor. Die Anzahl der Kopplungselemente 30a, 30b, 30c kann je nach Bedarf erhöht oder reduziert werden. Ferner ist die Form dieser Kopplungselemente 30a, 30b, 30c nicht auf die gezeigte quadratisch Variante beschränkt sondern kann auch in jeder weiteren denkbaren Form ausgestaltet sein. Weiterhin sind aus der Figur 3 die Verbindungselemente 28, 29 ersichtlich, die als Nut und Aussparung ausgebildet sein können.

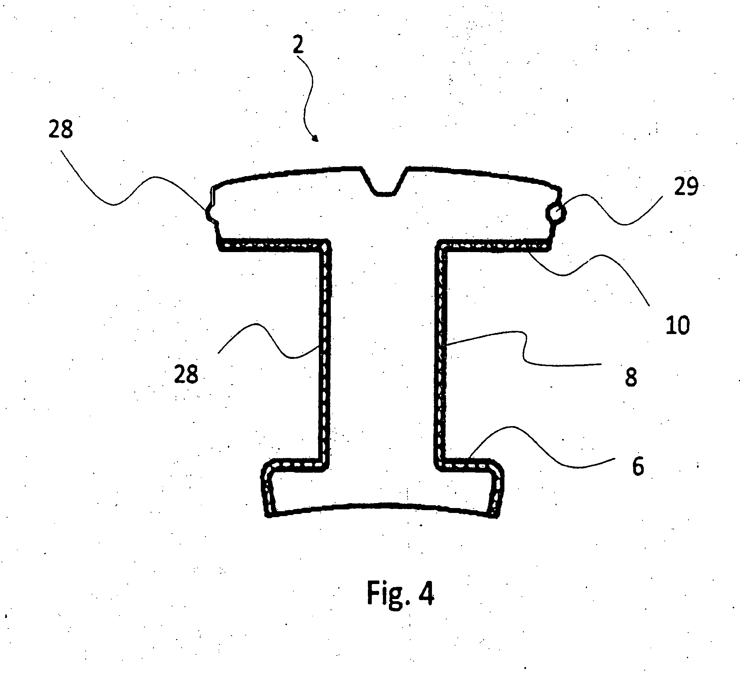

In Figur 4 ist eine Schnittdarstellung eines Statorzahnes 2 gezeigt. Die Isolationsschicht 32 deckt hierbei den ersten Abschnitt 8, den zweiten Abschnitt 6 und den dritten Abschnitt 10 zumindest teilweise ab. Es ist ersichtlich, dass die Isolationsschicht 32 kontinuierlich verläuft und daher keine Lücken aufweist.

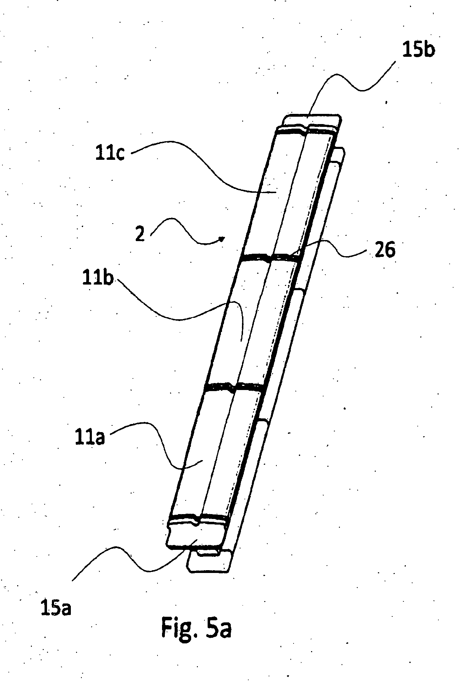

In Figur 5a ist ein aus mehreren Teilen zusammengesetzter Statorzahn 2 ersichtlich. Der Statorzahn 2 ist hierbei aus den Segmenten 11a, 11b, 11c und den beiden Endstücken 15a, 15 b zusammen gesetzt. Hierbei ist es möglich, dass lediglich zwei Statorsegmente 11a, 11 b oder mehr als drei Statorsegmente zusammengefügt werden.

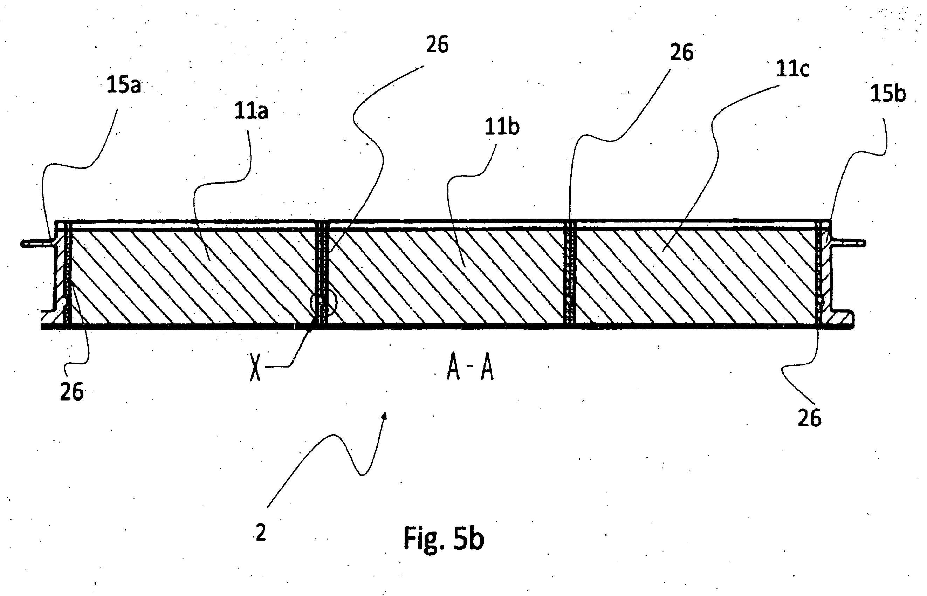

In Figur 5b ist eine Seitenansicht des zuvor beschriebenen Statorzahnes 2 gezeigt. Wie aus der Figur 5b ersichtlich wird, ist der Kontaktbereich 26 derart ausgelegt, dass beispielsweise das Segment 11 a an dem Segment 11 b und das Segment 11 b an dem Segment 11 c angeordnet werden kann. Ferner geht aus der Figur 5b hervor, dass die Endstücke 15a und 15b nicht identisch ausgestaltet sind, da das eine Endstück 15a in das Segment 11 a eingreift und das Segment 11 c in das Endstück 15b eingreift. Eine Anordnung mit identisch ausgestalteten Endstücken 15a, 15b ist jedoch auch vorstellbar.

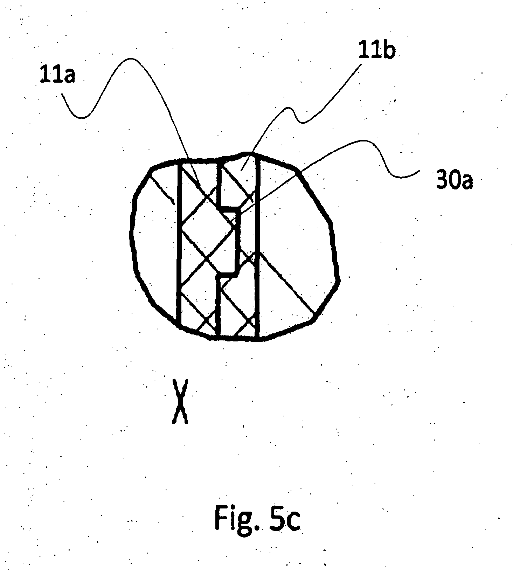

In Figur 5c zeigt ein vergrößerter Ausschnitt den in Figur 5b mit X gekennzeichneten Bereich, worin ein Kopplungselement 30a gezeigt ist. Hierbei greift Kopplungselement 30a des Segmentes 11a in eine Vertiefung des Elementes 11 b. Die Stelle an der die Kopplungselemente 30a, 30b, 30c vorgesehen sind kann beliebig gewählt werden, es ist lediglich wichtig, dass die Kopplungselemente 30a, 30b, 30c verschiedener Segmente 11 a, 11 b, 11 c bzw. Endstücke 15a, 15b kompatibel miteinander sind.

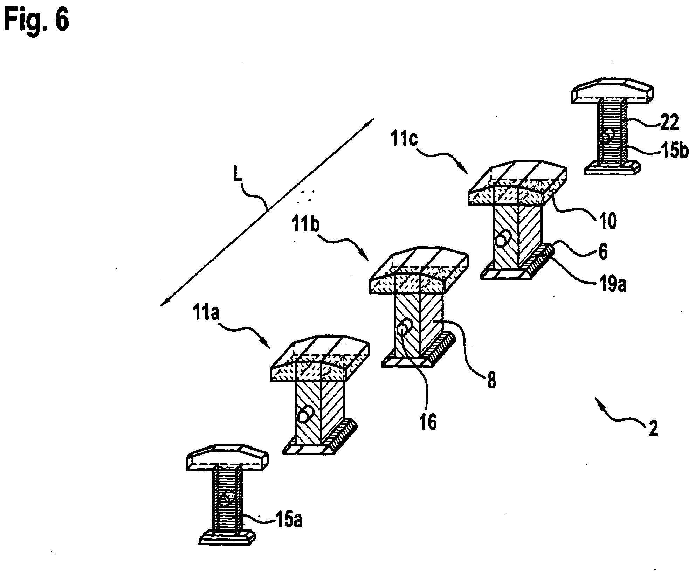

Figur 6 zeigt eine Explosionsdarstellung eines Statorzahnes 2. Wie aus der Figur 6 hervorgeht besteht der Statorzahn 2 in der gezeigten Variante aus zwei Endstücken 15a, 15b und drei Segmenten 11a, 11 b, 11c. Die Segmente 11a, 11 b, 11 c weisen hierbei jeweils einen ersten Abschnitt 8, einen zweiten Abschnitt 6 und einen dritten Abschnitt 10 auf. Der erste Abschnitt 8 aller Segmente 11a, 11 b, 11 c bildet einen Verbindungsbereich zwischen den Endstücken 15a, 15b.

Im Übergangsbereich vom ersten Abschnitt 8 zum zweiten Abschnitt 6 ist eine kontinuierliche Isolationsschicht 32 auf beiden Abschnitten aufgebracht. Der zweite Abschnitt 6 erstreckt sich in der Richtung orthogonal zur Längsachse L über den Verbildungsbereich hinaus. Der Teil des zweiten Abschnittes 6, der im diesem Übergangsbereich mit einer Kunststoffschicht bedeckt ist, erstreckt sich lediglich über einen Bruchteil der Tiefe des dritten Abschnittes 10. Der Übergangsbereich von ersten Bereich 8 zum dritten Bereich 10 kann entsprechend dem Übergangsbereich vom ersten Bereich zum zweiten Bereich gestaltet sein.

Hierbei ist die Projektion des dritten Abschnittes 10 der Segmente 11a, 11 b, 11 c auf eine Ebene größer als die entsprechende Projektion des zweiten Abschnittes 6 auf die Ebene. Der elektrisch isolierte Teil des dritten Abschnittes erstreckt in der Richtung orthogonal zu der Richtung der Längsachse L über den beschichteten Teil des zweiten Abschnittes 6 hinaus. Die Segmente 11 a, 11b, 11c weisen Kopplungselemente auf, die durch Fügetechnik miteinander verbunden werden. Es geht aus der Figur 2 hervor, dass das Segment 11 b zwischen den Segmenten 11a und 11c angeordnet ist und daher keinen Kontakt zu einem der Endstücke 15a, 15b aufweist. Weiter kann der Querschnitt der Endstücke 15a, 15b dem der Segmente 11 a, 11 b, 11 c entsprechen. Die Endstücke 15a, 15b weisen weiterhin einen gerillten Bauteilabschnitt 22 auf, der zur Drahtführung beim Wickeln der Spule dient. Die Endstücke 15a, 15b sind aus einem nichtleitenden Material gefertigt und die Länge eines Endstückes 15a, 15b entspricht dem Bruchteil der Länge eines Segmentes 11 a, 11b, 11c.

## Patentansprüche

1. Elektromotor mit wenigstens einem Stator und einem Rotor, wobei der Stator mindestens einen Statorzahn (2) aufweist und wenigstens ein Statorzahn (2) mindestens ein erstes und ein zweites Endstück (15a, 15b) wie auch mindestens einen die Endstücke (15a, 15b) verbindenden Verbindungkörper zwischen dem ersten und dem zweiten Endstück (15a, 15b) aufweist, wobei der Verbindungskörper mehrteilig ausgebildet ist und aus mindestens zwei Segmenten (11a, 11b) besteht, und wobei der Verbindungskörper mindestens einen ersten Abschnitt (8) als Anlagefläche für einen Teil einer Spule aufweist, wobei Kopplungselemente (30a, 30b, 30c) innerhalb der Kontaktbereiche (26) zwischen zwei Segmenten (11a, 11b) und zwischen Segmenten (11a, 11b) und den Endstücken (15a, 15b) vorgesehen sind, wobei die Endstücke (15a, 15b) aus Kunststoff bestehen und elektrisch isolierend ausgebildet sind, wobei wengistens der erste Abschnitt (8) elektrisch isolierend ausgebildet ist, **daduch gekennzeichnet, dass** der wenigstens erste Abschnitt (8) zumindest teilweise kunststoffumspritzt ist, und wobei eine Wärmeleitfähigkeit der Kunststoffumspritzung mehr als 1,0 W/Km beträgt.

2. Elektromotor nach Anspruch 1, **dadurch gekennzeichnet, dass** die Segmente (11a, 11b) unlösbar miteinander verbunden sind.

3. Elektromotor nach mindestens einem der vorangegangenen Ansprüche, **dadurch gekennzeichnet, dass** jedes Endstück (15a, 15b) unlösbar mit einem Segment (11a, 11b) verbunden ist.

4. Elektromotor nach mindestens einem der vorangegangenen Ansprüche, **dadurch gekennzeichnet, dass** der erste Abschnitt (8) eine Länge (L) von mindestens 10 mm, bevorzugt von 50-90 mm und bevorzugt eine Länge in Abhängigkeit von einem Vielfachen der Magnetlänge, aufweist.

5. Elektromotor nach mindestens einem der vorangegangenen Ansprüche, **dadurch gekennzeichnet, dass** der erste Abschnitt (8) eine Kunststoffschicht aufweist, deren Dicke unter 0,5 mm, bevorzugt unter 0,4 mm und besonders bevorzugt unter 0,3 mm liegt.

6. Elektromotor nach mindestens einem der vorangegangenen Ansprüche, **dadurch gekennzeichnet, dass** innerhalb der Segmente magnetisierbare Körper angeordnet sind.

7. Elektromotor nach mindestens einem der vorangegangenen Ansprüche, **dadurch gekennzeichnet, dass** der Querschnitt der Endstücke (15a, 15b) dem Querschnitt der Segmente (11a, 11b) entspricht.

8. Verfahren zum Herstellen eines Statorzahnes (2) für einen Elektromotor gemäß einem der vorhergehenden Ansprüche, wobei der Statorzahn (2) einen ersten Abschnitt (8) als Anlagefläche für einen Teil einer Spule aufweist, bei welchem der erste Abschnitt (8) aus mindestens zwei Segmenten (11a, 11b) hergestellt wird, die in der Längsrichtung (L) eines Statorzahns (2) angeordnet werden, wobei die Segmente (11a, 11b) von einem ersten (15a) und einem zweiten Endteil (15b) in Längsrichtung (L) abgeschlossen werden, wobei Kopplungselemente (30a, 30b, 30c) innerhalb der Kontaktbereiche (26) zwischen zwei Segmenten (11a, 11b) und zwischen Segmenten (11a, 11b) und den Endstücken (15a, 15b) vorgesehen werden, wobei die Endstücke (15a, 15b) aus Kunststoff bestehen und elektrisch isolierend ausgebildet ist, wobei wenigstens der erste Abschnitt (8) elektrisch isolierend ausgebildet ist, **dadurch gekennzeichnet, dass** der wenigstens erste Abschnitt (8) zumindest teilweise kunststoffumspritzt ist, und wobei eine Wärmeleitfähigkeit der Kunststoffumspritzung mehr als 1,0 W/Km beträgt.

## Claims

1. Electric motor comprising at least one stator and one rotor, wherein the stator has at least one stator tooth (2), and at least one stator tooth (2) has at least one first and one second end piece (15a, 15b) as well as at least one connecting body between the first and second end pieces (15a, 15b) which connects the end pieces (15a, 15b), wherein the connecting body has a multipart design and consists of at least two segments (11a, 11b), and wherein the connecting body has at least one first section (8) as resting surface for part of a coil, wherein clutch elements (30a, 30b, 30c) are provided within the contact regions (26) between two segments (11a, 11b) and between segments (11a, 11b) and the end pieces (15a, 15b), wherein the end pieces (15a, 15b) consist of plastic and are electrically insulating, wherein at least the first section (8) is electrically insulating, **characterized in that** at least the first section (8) is at least partially encapsulated by plastic injection moulding, and wherein a thermal conductivity of the plastic encapsulation is more than 1.0 W/mK.

2. Electric motor according to Claim 1, **characterized in that** the segments (11a, 11b) are connected nondetachably to one another.

3. Electric motor according to at least one of the preceding claims, **characterized in that** each end piece (15a, 15b) is connected nondetachably to a segment (11a, 11b).

4. Electric motor according to at least one of the preceding claims, **characterized in that** the first section (8) has a length (L) of at least 10 mm, preferably of 50-90 mm and preferably a length depending on a multiple of the magnet length.

5. Electric motor according to at least one of the preceding claims, **characterized in that** the first section (8) has a plastic layer with a thickness of below 0.5 mm, preferably below 0.4 mm and particularly preferably below 0.3 mm.

6. Electric motor according to at least one of the preceding claims, **characterized in that** magnetizable bodies are arranged within the segments.

7. Electric motor according to at least one of the preceding claims, **characterized in that** the cross section of the end pieces (15a, 15b) corresponds to the cross section of the segments (11a, 11b).

8. Method for producing a stator tooth (2) for an electric motor according to one of the preceding claims, wherein the stator tooth (2) has a first section (8) as resting surface for part of a coil, in which method the first section (8) is produced from at least two segments (11a, 11b), which are arranged in the longitudinal direction (L) of a stator tooth (2), wherein the segments (11a, 11b) are terminated by a first end part (15a) and a second end part (15b) in the longitudinal direction (L), wherein clutch elements (30a, 30b, 30c) are provided within the contact regions (26) between two segments (11a, 11b) and between segments (11a, 11b) and the end pieces (15a, 15b), wherein the end pieces (15a, 15b) consist of plastic and are electrically insulating, wherein at least the first section (8) is electrically insulating, **characterized in that** at least the first section (8) is at least partially encapsulated by plastic injection moulding, and wherein a thermal conductivity of the plastic encapsulation is more than 1.0 W/mK.

## Revendications

1. Moteur électrique comprenant au moins un stator et un rotor, le stator présentant au moins une dent de stator (2) et au moins une dent de stator (2) présentant au moins un premier et un deuxième bout (15a, 15b) ainsi qu'au moins un corps de liaison reliant les bouts (15a, 15b) entre le premier et le deuxième bout (15a, 15b), le corps de liaison étant réalisé en plusieurs parties et se composant d'au moins deux segments (11a, 11b), et le corps de liaison présentant au moins une première portion (8) en tant que surface de butée pour une partie d'une bobine, des éléments d'accouplement (30a, 30b, 30c) étant prévus à l'intérieur des régions de contact (26) entre deux segments (11a, 11b) et entre des segments (11a, 11b) et les bouts (15a, 15b), les bouts (15a, 15b) étant constitués de plastique et étant réalisés de manière électriquement isolante, au moins la première portion (8) étant formée de manière électriquement isolante, **caractérisé en ce que** l'au moins une première portion (8) est au moins en partie surmoulée de plastique, et une conductivité thermique du surmoulage de plastique étant supérieure à 1,0 W/Km.

2. Moteur électrique selon la revendication 1, **caractérisé en ce que** les segments (11a, 11b) sont connectés les uns aux autres de manière indissociable.

3. Moteur électrique selon au moins l'une quelconque des revendications précédentes, **caractérisé en ce que** chaque bout (15a, 15b) est connecté de manière indissociable à un segment (11a, 11b).

4. Moteur électrique selon au moins l'une quelconque des revendications précédentes, **caractérisé en ce que** la première portion (8) présente une longueur (L) d'au moins 10 mm, de préférence de 50-90 mm et de préférence une longueur en fonction d'un multiple de la longueur de l'aimant.

5. Moteur électrique selon au moins l'une quelconque des revendications précédentes, **caractérisé en ce que** la première portion (8) présente une couche de plastique dont l'épaisseur est inférieure à 0,5 mm, de préférence inférieure à 0,4 mm, et particulièrement préférablement inférieure à 0,3 mm.

6. Moteur électrique selon au moins l'une quelconque des revendications précédentes, **caractérisé en ce qu'**à l'intérieur des segments sont disposés des corps magnétisables.

7. Moteur électrique selon au moins l'une quelconque des revendications précédentes, **caractérisé en ce que** la section transversale des bouts (15a, 15b) correspond à la section transversale des segments (11a, 11b).

8. Procédé de fabrication d'une dent de stator (2) pour un moteur électrique selon l'une quelconque des revendications précédentes, dans lequel la dent de stator (2) présente une première portion (8) en tant que surface de butée pour une partie d'une bobine, dans lequel la première portion (8) est fabriquée à partir d'au moins deux segments (11a, 11b) qui sont disposés dans la direction longitudinale (L) d'une dent de stator (2), les segments (11a, 11b) étant terminés par un premier (15a) et un deuxième (15b) bout dans la direction longitudinale (L), des éléments d'accouplement (30a, 30b, 30c) étant prévus à l'intérieur des régions de contact (26) entre deux segments (11a, 11b) et entre des segments (11a, 11b) et les bouts (15a, 15b), les bouts (15a, 15b) étant constitués de plastique et étant réalisés de manière électriquement isolante, au moins la première portion (8) étant formée de manière électriquement isolante, **caractérisé en ce que** l'au moins une première portion (8) est au moins en partie surmoulée de plastique, et une conductivité thermique du surmoulage de plastique étant supérieure à 1,0 W/Km.
